# EUROPEAN PATENT SPECIFICATION

(11) **EP 3 182 131 B1**
(45) Date of publication and mention of the grant of the patent: **18.09.2019**
(21) Application number: 16204763.3
(22) Date of filing: 16.12.2016
(51) Int. Cl.: G01N 33/82

(54) **SEPARATING AND QUANTIFYING THIAMINE PYROPHOSPHATE AND PYRIDOXAL 5-PHOSPHATE IN HUMAN WHOLE BLOOD**
TRENNUNG UND QUANTIFIZIERUNG VON THIAMINPYROPHOSPHAT UND PYRIDOXAL-5-PHOSPHAT IN MENSCHLICHEM VOLLBLUT
SÉPARATION ET QUANTIFICATION DE THIAMINE PYROPHOSPHATE ET DE 5-PHOSPHATE DE PYRIDOXAL DANS LE SANG HUMAIN TOTAL

(30) Priority: 16.12.2015 US 201562268190 P
(43) Date of publication of application: 21.06.2017
(73) Proprietor: Micromass UK Limited, Wilmslow SK9 4AX (GB)
(72) Inventor: Wardle, Robert M., Manchester M34 6FQ (GB); Calton, Lisa Jane, Stockport SK7 4JA (GB)
(74) Representative: Barton, Matthew Thomas

(56) References cited:
- WO-A1-2013/010553
- US-A1- 2009 203 145
- JOHAN PUTS ET AL: "Simultaneous Determination of Underivatized Vitamin B1 and B6 in Whole Blood by Reversed Phase Ultra High Performance Liquid Chromatography Tandem Mass Spectrometry", PLOS ONE, vol. 10, no. 7, 2 July 2015 (2015-07-02), page e0132018, XP055354332, DOI: 10.1371/journal.pone.0132018

## Description

### REFERENCE TO RELATED APPLICATIONS

### TECHNICAL FIELD

The present disclosure generally relates to methods for separating and quantifying Thiamine Pyrophosphate (TPP, Vitamin B1) and Pyridoxal 5-Phosphate (PLP, Vitamin B6) from human whole blood.

### BACKGROUND

Vitamin B1 (Thiamine) is a water soluble coenzyme that has a role in nervous function, the metabolism of carbohydrates and fatty acids, and is vital for normal growth and development. Thiamine is metabolized by Thiamine Pyrophosphokinase + ATP into its physiologically active form, Thiamine Pyrophosphate (TPP). Vitamin B6 is a group of three vitamins; Pyridoxal, Pyridoxine and Pyridoxamine. The biologically active form of Vitamin B6 is Pyridoxal 5-Phosphate (PLP), a coenzyme that is involved in approximately one hundred enzymatic reactions, including the synthesis and breakdown of amino acids, phospholipids and glycogen.

The analysis of both PLP and TPP is important to diagnose potential nutritional deficiency which often occurs in elderly people due to malnutrition, in severe alcoholism, and in gastrointestinal compromise due to surgery or disease. This can lead to conditions such as e.g., Beriberi, Wernicke-Korsakoff syndrome, alterations of the skin, neurological disorders, anemias, and increases in homocysteine (hyperhomocysteinemia). PLP and TPP have traditionally been analyzed independently using high performance liquid chromatography (HPLC) consisting of separate HPLC methods where total run times for each method can be up to 20 min long. In addition, pre-column derivatization of the PLP and TPP, such as with fluorescence-based detection, is also usually required. These methods are not realistic in a high-throughput clinical setting because they are laborious, costly, and typically require the use of toxic reagents such as potassium ferricyanide.

Simultaneous Determination of Underivatized Vitamin B1 and B6 in Whole Blood by Reversed Phase Ultra High Performance Liquid Chromatography Tandem Mass Spectrometry (J. Puts et al., PLOS One, 2 July 2015, 10(7)) relates to the simultaneous determination of underivatized vitamin B1 and B6 in whole blood by reversed ultra high performance liquid chromatography tandem mass spectrometry.

US 2009/0203145 A1 relates to methods for determining the level of B vitamins in a test sample e.g., infant formula.

### SUMMARY

The present invention provides a method of quantifying both native Thiamine Pyrophosphate (TPP) and native Pyridoxal 5-Phosphate (PLP) from a single sample of human whole blood using mass spectrometry, comprising: eluting TPP and PLP at time of less than 20 seconds apart from one another from a liquid chromatography column; generating a mass spectrometry signal during elution of the TPP and PLP; and quantifying the TPP and PLP using one or more calibration standards.

Preferably, quantifying the TPP and PLP using one or more calibration standards comprises augmenting human whole blood with TPP and/or PLP, and optionally diluting the human whole blood with phosphate buffered saline to generate the one or more calibration standards over a measuring range to which the amount of the TPP and PLP eluted from the column can be determined by mathematical expression.

Preferably, quantifying the TPP and PLP using one or more calibration standards comprises augmenting TPP and PLP depleted whole blood with TPP and PLP to generate the one or more calibration standards over a measuring range to which the amount of the TPP and PLP eluted from the column can be determined by mathematical expression.

Preferably, quantifying the TPP and PLP using one or more calibration standards comprises the use of surrogate matrix calibrators prepared from phosphate buffered saline solution optionally containing human serum albumin or bovine serum albumin and optionally augmented with TPP and PLP to generate the one or more calibration standards over a measuring range to which the amount of the TPP and PLP eluted from the column can be determined by mathematical expression.

Preferably, quantifying the TPP and PLP using one or more calibration standards comprises the use of surrogate analyte calibrators prepared in human whole blood by the addition of a different stable labeled isotope to that of the internal standard for TPP and PLP to generate the one or more calibration standards over a measuring range to which the amount of the TPP and PLP eluted from the column can be determined by mathematical expression.

Preferably, quantifying the TPP and PLP using one or more calibration standards comprises the use placing one or more calibration standards in the same sample together with the TPP and PLP eluted from the column, to which the amount of the TPP and PLP eluted from the column can be determined by mathematical expression.

Preferably, the one or more calibrators are provided in the form of a kit.

Also provided is a method of separating both native Thiamine Pyrophosphate (TPP) and native Pyridoxal 5-Phosphate (PLP) from whole blood comprising eluting TPP and PLP at time of less than 20 seconds apart from one another from a liquid chromatography column.

Preferably, eluting TPP and PLP at time of less than 17 seconds apart from one another from the liquid chromatography column.

Preferably, eluting TPP and PLP at time of less than 15 seconds apart from one another from the liquid chromatography column.

Preferably, eluting TPP and PLP at time of less than 13 seconds apart from one another from the liquid chromatography column.

Preferably, both the TPP and PLP have a retention time on the liquid chromatography column of 35 seconds or greater.

Preferably, both the TPP and PLP have a retention time on the liquid chromatography column of 40 seconds or greater.

Preferably, both the TPP and PLP have a retention time on the liquid chromatography column of 45 seconds or greater.

Preferably, the total elution time of TPP and PLP does not exceed 3.5 minutes.

Preferably, the total elution time of TPP and PLP does not exceed 3.0 minutes.

Preferably, the total elution time of TPP and PLP does not exceed 2.5 minutes.

Preferably, the total elution time of TPP and PLP does not exceed 2.0 minutes.

Preferably, the liquid chromatography column is a reverse phase C18 HPLC column.

Preferably, the liquid chromatography column is a reverse phase C18 HPLC column having a 3.5 µm particle size.

Preferably, the liquid chromatography column is a reverse phase C18 HPLC column having a 3.5 µm particle size, a greater than 15% carbon load, and a pore size of greater than or equal to 100 Å.

Preferably, the liquid chromatography column is a reverse phase C18 HPLC column having a 3.5 µm particle size, a 15% to 20% carbon load, and a pore size of 100Å.

Preferably, the TPP and PLP are eluted from the liquid chromatography column using a mobile phase comprising water (H₂O), methanol (MeOH) and formic acid.

Preferably, the TPP and PLP are eluted from the liquid chromatography column using a mobile phase comprising H₂O together with 0.1% formic acid (v/v) and MeOH together with 0.1% formic acid (v/v).

Preferably, the TPP and PLP are eluted from the liquid chromatography column using H₂O together with 0.1% formic acid (v/v) and MeOH together with 0.1% formic acid (v/v), subject to a gradient comprising the following conditions:

| Time (min) | Flow Rate (mL/min) | % Total of H₂O together with 0.1% formic acid (v/v) | % Total of MeOH together with 0.1% formic acid (v/v) | Curve |
|---|---|---|---|---|
| 0 | 0.60 | 97 | 3 | Initial |
| 0.60 | 0.60 | 70 | 30 | 6 |
| 1.20 | 0.80 | 3 | 97 | 11 |
| 1.70 | 0.80 | 97 | 3 | 11 |
| 1.90 | 0.60 | 97 | 3 | 11 |

Preferably, including the step of protein precipitating the TPP and PLP from whole blood prior to elution on the liquid chromatography column.

Preferably, prior to eluting TPP and PLP, the TPP and PLP are mixed with trichloroacetic acid.

In general, the present disclosure relates to robust, high-throughput, and clinically applicable methods for simultaneously separating and quantifying the biologically active forms of Vitamin B1 (TPP) and Vitamin B6 (PLP) from human whole blood.

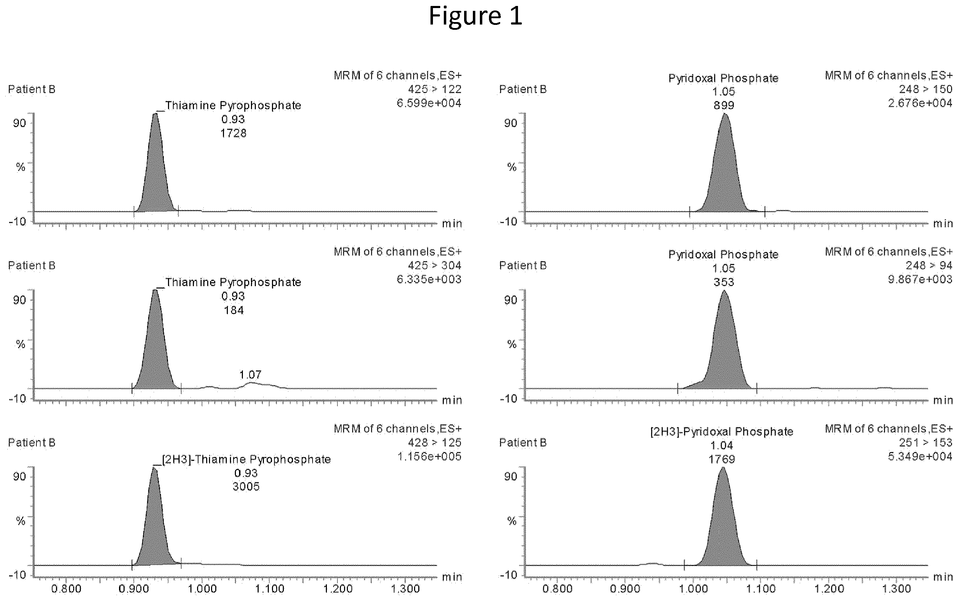

In one aspect, the disclosed methods comprise a single HPLC run from a sample of human whole blood without the need for pre-column derivatization. See e.g., **Figure 1****.** Thus, in one aspect, the methods disclosed herein eliminate the need for multiple HPLC runs and the use of toxic derivatization reagents.

In another aspect, the methods described herein minimize the elution time between the TPP and PLP on the chromatography column (e.g., less than 20 seconds apart from one another). See e.g., **Figure 1****.** Thus, in one aspect, this allows most of the column waste to be diverted from the mass spectrometer (e.g., in the case of quantification), thereby minimizing effects from early eluting interferences, which, as a result, improves detection sensitivity and accuracy as well as method robustness.

In yet another aspect, the methods described herein comprise short run times (e.g., total run times of 3.5 minutes or less, such as 3.2 minutes or less) and optimized retention times for TPP and PLP (e.g., tR of 45 seconds or greater. See e.g., **Figure 1****.** Thus, in one aspect, this significantly reduces the amount of solvents used, lowers cost(s), and allows for a high throughput of samples to be analyzed.

In yet another aspect, the methods described herein implement in certain aspects a mass spectrometer analysis of TPP and PLP where there is a single sample including a first known quantity of TPP and a first known quantity of PLP, and where the first known quantity of TPP, the known quantity of PLP, and the PLP and TPP from simultaneous separation from whole blood are each distinguishable within the single sample by mass spectrometry. Thus, in one aspect, this greatly reduces analysis times and eliminates the inefficiency of conventional calibration that uses multiple calibrators independently. See e.g., WO 2012/170549.

### BRIEF DESCRIPTION OF THE FIGURES

**Figure 1** depicts HPLC chromatograms of the simultaneous separation of PLP and TPP using the methods described herein.
**Figure 2** illustrates the signal to noise ratio of TPP and PLP from a low patient sample using the methods described herein.
**Figure 3** illustrates the calibration lines obtained from whole blood calibrators using the methods described herein.

### DETAILED DESCRIPTION OF CERTAIN EMBODIMENTS

In a first exemplary embodiment, provided is a method of quantifying both native Thiamine Pyrophosphate (TPP) and native Pyridoxal 5-Phosphate (PLP) from a single sample of human whole blood using mass spectrometry, comprising eluting TPP and PLP at time of less than 20 seconds apart from one another from a liquid chromatography column; generating a mass spectrometry signal during elution of the TPP and PLP; and quantifying the TPP and PLP using one or more calibration standards.

As used herein, the terms "native Thiamine Pyrophosphate (TPP)" and "native Pyridoxal 5-Phosphate (PLP)" mean TPP and PLP which have not been subjected to or undergone pre-column derivatization. It is to be understood that pre-column derivatization refers to any methods that are employed prior to analysis which chemically modify TPP, PLP, or both. Thus, sample preparation methods which does not involve chemical modification of TPP and PLP (e.g., precipitation, dilution, extraction, filtering, centrifugation, drying, and the like) do not constitute pre-column derivatization methods.

As used herein, calibration standards, also referred to as calibrators, mean a standard or reference material that contains a known amount of TPP or PLP, or both. These known amounts can then be used to generate a calibration line to which the amount of TPP and/or PLP can be determined from a sample containing an unknown amount. The use of calibration standards to determine unknown concentrations is known in the art. For example, for each calibrator standard the ratio of the peak area of TPP to the peak area of its corresponding internal standard is calculated. The determined response ratio (y) is plotted against the calibrator standard concentration (x) for TPP. A line of best fit (this can be linear, or quadratic) is drawn through all the calibrators and the equation of the line is determined, for example a linear line of best fit equation: y = 0.00619507 ^{∗} x + 0.0442534. Using the determined equation of the calibration line, the TPP concentration in an unknown sample (x) can be calculated by rearranging the equation: x = (y - 0.0442534) ÷ 0.00619507 and determining the response ratio in the unknown sample (for example y = 0.6). Therefore, x = (0.6 - 0.0442534) ÷ 0.00619507; x = 89.7. Thus, the concentration of the unknown sample is 89.7nmol/L. The concentration of PLP is determined e.g., in the same manner.

It is to be understood that while not mentioned in detail, other methods for determining the amount of TPP and/or PLP from a sample containing an unknown amount using calibration standards are encompassed. Thus, such determinations are not limited to those that require the use of mathematical equations. For example, unknown amounts can be determined by simply reading the response ratios alone, by reading the graph or calibrations lines, or the like.

Calibrators used by the methods described herein can be prepared using a number of matrices, e.g., by i) human whole blood augmented with TPP and PLP and/or diluted with 0.01M phosphate buffered saline solution (pH 7.4), which may contain 1% human serum albumin or 1% bovine serum albumin to generate the calibrators over a desired measuring range; by ii) TPP and PLP depleted whole blood augmented with TPP and PLP to generate the calibrators over a desired measuring range; by iii) surrogate matrix calibrators prepared from 0.01M phosphate buffered saline solution (pH 7.4) which may contain 1% human serum albumin or 1% bovine serum albumin and augmented with TPP and PLP to generate the calibrators over a desired measuring range; by iv) surrogate analyte calibrators prepared in human whole blood by the addition of a different stable labeled isotope to that of the internal standard for TPP and PLP to generate a surrogate analyte calibration curve for TPP and PLP over the desired measuring range; or by v) a single sample approach where one or more calibration standards (each of which having a different mass and/or fragmentation pattern) are contained in the same sample as the TPP and PLP and are eluted from the liquid chromatography column. See e.g., WO 2012/170549.

Thus, in a second exemplary embodiment, quantifying the TPP and PLP using one or more calibration standards comprises augmenting human whole blood with TPP and/or PLP, and optionally diluting the human whole blood with phosphate buffered saline to generate the one or more calibration standards over a measuring range to which the amount of the TPP and PLP eluted from the column can be determined by mathematical expression, wherein the remaining features are as described in the first exemplary embodiment. Alternatively, quantifying the TPP and PLP using one or more calibration standards comprises augmenting TPP and PLP depleted whole blood with TPP and PLP to generate the one or more calibration standards over a measuring range to which the amount of the TPP and PLP eluted from the column can be determined by mathematical expression, wherein the remaining features are as described in the first exemplary embodiment. In another alternative, quantifying the TPP and PLP using one or more calibration standards comprises the use of surrogate matrix calibrators prepared from phosphate buffered saline solution optionally containing human serum albumin or bovine serum albumin and optionally augmented with TPP and PLP to generate the one or more calibration standards over a measuring range to which the amount of the TPP and PLP eluted from the column can be determined by mathematical expression, wherein the remaining features are as described in the first exemplary embodiment. In another alternative, quantifying the TPP and PLP using one or more calibration standards comprises the use of surrogate analyte calibrators prepared in human whole blood by the addition of a different stable labeled isotope to that of the internal standard for TPP and PLP to generate the one or more calibration standards over a measuring range to which the amount of the TPP and PLP eluted from the column can be determined by mathematical expression, wherein the remaining features are as described in the first exemplary embodiment. In yet another alternative, quantifying the TPP and PLP using one or more calibration standards comprises the placing one or more calibration standards of different masses and/or fragmentation patters in the same sample following the methods described in WO 2012/170549 together with the TPP and PLP eluted from the column, to which the amount of the TPP and PLP eluted from the column can be determined by e.g., mathematical expression, wherein the remaining features are as described in the first exemplary embodiment. Each of the calibrators described above may be provided in the form of a kit together with instructions to use said kit. Also, other non-mathematical based determinations for each of the above alternatives are contemplated and include e.g., reading the response ratios alone, reading the graph or calibrations lines, or the like.

Mathematical determinations of the amount of TPP and PLP eluted from the column when compared with the one or more calibrators described herein (such as the in the first and second exemplary embodiment) can be made following the methods described in e.g., WO 2012/170549. Again, other non-mathematical based determinations are contemplated and include e.g., reading the response ratios alone, reading the graph or calibrations lines, or the like.

In an third exemplary embodiment, also provided is a method of separating both native Thiamine Pyrophosphate (TPP) and native Pyridoxal 5-Phosphate (PLP) from whole blood comprising eluting TPP and PLP at time of less than 20 seconds apart from one another from a liquid chromatography column.

In a fourth exemplary embodiment, the methods described in the first, second, or third exemplary embodiment comprises eluting TPP and PLP at time of less than 17 seconds apart from one another from the liquid chromatography column. Alternatively, the methods described in the first, second, or third exemplary embodiment comprises eluting TPP and PLP at time of less than 15 seconds apart from one another from the liquid chromatography column. In another alternative, the methods described in the first, second, or third exemplary embodiment comprises eluting TPP and PLP at time of less than 13 seconds apart from one another from the liquid chromatography column.

In a fifth exemplary embodiment, both the TPP and PLP have a retention time on the liquid chromatography column of 35 seconds or greater, wherein the remaining features are as described in the first, second, third, or fourth exemplary embodiment. Alternatively, both the TPP and PLP have a retention time on the liquid chromatography column of 40 seconds or greater, wherein the remaining features are as described in the first, second, third, or fourth exemplary embodiment. In another alternative, both the TPP and PLP have a retention time on the liquid chromatography column of 45 seconds or greater, wherein the remaining features are as described in the first, second, third, or fourth exemplary embodiment.

In a sixth exemplary embodiment, the total elution time of TPP and PLP does not exceed 3.5 minutes, wherein the remaining features are as described in the first, second, third, fourth, or fifth exemplary embodiment. Alternatively, the total elution time of TPP and PLP does not exceed 3.2 minutes, wherein the remaining features are as described in the first, second, third, fourth, or fifth exemplary embodiment. In another alternative, the total elution time of TPP and PLP does not exceed 3.0 minutes, wherein the remaining features are as described in the first, second, third, fourth, or fifth exemplary embodiment. In another alternative, the total elution time of TPP and PLP does not exceed 2.5 minutes, wherein the remaining features are as described in the first, second, third, fourth, or fifth exemplary embodiment. In yet another alternative, the total elution time of TPP and PLP does not exceed 2.0 minutes, wherein the remaining features are as described in the first, second, third, fourth, or fifth exemplary embodiment.

In a seventh exemplary embodiment, the liquid chromatography column is a reverse phase C18 HPLC column, wherein the remaining features are as described in the first, second, third, fourth, fifth, or sixth embodiment.

In an eighth exemplary embodiment, the liquid chromatography column is a reverse phase C18 HPLC column having a 3.5 µm particle size, wherein the remaining features are as described in the first, second, third, fourth, fifth, sixth, or seventh exemplary embodiment.

In a ninth exemplary embodiment, the liquid chromatography column is a reverse phase C18 HPLC column having a 3.5 µm particle size, a greater than 15% carbon load, and a pore size of greater than or equal to 100 Å, wherein the remaining features are as described in the first, second, third, fourth, fifth, sixth, seventh, eighth, or ninth exemplary embodiment.

In a tenth exemplary embodiment, the liquid chromatography column is a reverse phase C18 HPLC column having a 3.5 µm particle size, a 15% to 20% carbon load, and a pore size of 100Å, wherein the remaining features are as described in the first, second, third, fourth, fifth, sixth, seventh, eighth, or ninth exemplary embodiment.

In an eleventh exemplary embodiment, the TPP and PLP are eluted from the liquid chromatography column using a mobile phase comprising water (H₂O), methanol (MeOH) and formic acid, wherein the remaining features are as described in the first, second, third, fourth, fifth, sixth, seventh, eighth, ninth, or tenth embodiment.

In a twelfth exemplary embodiment, the TPP and PLP are eluted from the liquid chromatography column using a mobile phase comprising H₂O together with 0.1% formic acid (v/v) and MeOH together with 0.1% formic acid (v/v), wherein the remaining features are as described in the first, second, third, fourth, fifth, sixth, seventh, eighth, ninth, tenth, or eleventh exemplary embodiment.

In a thirteenth exemplary embodiment, the TPP and PLP are eluted from the liquid chromatography column using H₂O together with 0.1% formic acid (v/v) and MeOH together with 0.1% formic acid (v/v), subject to a gradient comprising the following conditions:

| Time (min) | Flow Rate (mL/min) | % Total of H₂O together with 0.1% formic acid (v/v) | % Total of MeOH together with 0.1% formic acid (v/v) | Curve |
|---|---|---|---|---|
| 0 | 0.60 | 97 | 3 | Initial |
| 0.60 | 0.60 | 70 | 30 | 6 |
| 1.20 | 0.80 | 3 | 97 | 11 |
| 1.70 | 0.80 | 97 | 3 | 11 |
| 1.90 | 0.60 | 97 | 3 | 11 |

wherein the remaining features are as described in the first, second, third, fourth, fifth, sixth, seventh, eighth, ninth, tenth, eleventh, or twelfth exemplary embodiment.

In a fourteenth exemplary embodiment, the method described herein such as those described in the first, second, third, fourth, fifth, sixth, seventh, eighth, ninth, tenth, eleventh, twelfth, or thirteenth exemplary embodiment, further comprises the step of protein precipitating the TPP and PLP from whole blood prior to elution on the liquid chromatography column.

### EXEMPLIFICATION

### Separation and Quantification of TPP and PLP Simultaneously from a Sample of Human Whole Blood

### Equipment/Materials

ACQUITY UPLC I-Class Binary Solvent Manager
ACQUITY UPLC I-Class Sample Manager with Flow Through Needle
ACQUITY UPLC I-Class Active Column Heater
Xevo TQD

### Methods

The LC required approximately 100 injections of a prepared whole blood sample to condition the column. Peaks were not visible in the first 5-10 samples for TPP or PLP and then their areas increased over the course of the injections. This was accommodated for when a new column was placed onto the system.

Mobile Phase A: H₂O + 0.1% Formic Acid
Mobile Phase B: MeOH + 0.1% Formic Acid
Purge Solvent: H₂O + 0.1% Formic Acid
Seal Wash: 80:20 H₂O:MeOH
Wash Solvent: 80:20 H₂O:MeOH
Column: Symmetry C18, 2.1 x 100mm, 3.5µm (P/N WAT058965)

**Gradient Table**

| **Time (min)** | **Flow Rate (mL/min)** | **%A** | **%B** | **Curve** |
|---|---|---|---|---|
| 0.00 | 0.60 | 97 | 3 | Initial |
| 0.60 | 0.60 | 70 | 30 | 6 |
| 1.20 | 0.80 | 3 | 97 | 11 |
| 1.70 | 0.80 | 97 | 3 | 11 |
| 1.90 | 0.60 | 97 | 3 | 11 |

| | |
|---|---|
| Inlet runtime: 2.0min | Low Pressure Limit: 1000psi |
| Seal wash: 1.5min | High Pressure Limit: 18000psi |

### Autosampler Conditions

Needle volume: 30µL (Optional extension loop)
Injection volume: 20µL (Or range of 5 to 50µL is also acceptable dependent upon mass spectrometer used)
Sample syringe volume: 100µL (Optional larger volume syringe)
Sample temperature: 10°C
Runtime: 2.0min
Column temperature: 30°C ± 2°C alarm
Load Ahead: Disabled
Needle placement: 2mm (when transferred or 10mm when injecting straight off pellet)

### Xevo TQD Parameters

Approximate (optimized upon tuning)
Capillary: 1.0kV (optimized upon tuning)
Source temperature: 150°C (A range of 120 to 170°C is also acceptable)
Desolvation temperature: 450°C (A range of 300 to 600°C is also acceptable)
Desolvation gas flow: 1000L/Hr
Extractor: 3V
RF lens: 2.5V
Cone gas flow: 10L/Hr
Inter-Scan Delay
MS inter-scan delay: 0.02sec
Polarity/mode switch inter-scan delay: 0.02sec
Inter-channel delay: 0.01sec

### MRM Transitions

**Approximate (optimized upon tuning)**

| **Compound** | **Precursor (m/z)** | **Product (m/z)** | **Cone (V)** | **Collision (kV)** |
|---|---|---|---|---|
| PLP (Quan) | 248.0 | 150.0 | 20* | 14 |
| PLP (Qual) | 248.0 | 94.0 | 20* | 28 |
| [²H₃]-PLP | 251.0 | 153.0 | 20* | 14 |
| TPP (Quan) | 425.0 | 122.0 | 30 | 22 |
| TPP (Qual) | 425.0 | 304.0 | 30 | 16 |
| [²H₃]-TPP | 428.0 | 125.0 | 30 | 22 |

| | | | | |
|---|---|---|---|---|
| *Cone Voltage not optimized due to an interfering compound at higher cone voltages. Value set to 20V | | | | |

The quantifier ions typically give better peak response, however other qualifier ions may be selected if deemed more suitable.

Dwell time for all compounds: 0.025sec (Auto Dwell not selected)

The solvent delay and divert settings allow for the first 0.75min of the run and the column wash to be diverted to waste. Given the higher flow rate of 0.6mL/min, this allows for a good portion of the early eluting interferences and those eluting in the column wash to not enter the MS system. After the column wash, the mobile phase flow is sent back into the MS system to wash any TCA that may still be present in the MS system.

| Time (min) | Event | Action |
|---|---|---|
| 0.00 | Flow State | Waste |
| 0.00 | Solvent Delay | Begin |
| 0.75 | Solvent Delay | End |
| 0.75 | Flow State | LC |
| 1.35 | Flow State | Waste |
| 1.35 | Solvent Delay | Begin |
| 1.75 | Flow State | LC |

Scan time: 2.0min

### Sample Preparation

50uL of internal standard working solution (approx 200ng/mL (470nmol/L) of [²H₃]-Thiamine Pyrophosphate & 100ng/mL (400nmol/L) of [²H₃]-Pyridoxal 5-Phosphate) was aliquoted into a 2mL Square 96-Well Plate.

While mixing the plate, 50µL of whole blood sample was added and mixing was left for at least 30min*. In one alternative, while mixing the plate, 50µL of whole blood sample was added and mixing was left for at least 30seconds.

While continuing to mix the plate, 400µL of 200g/L (20% (w/v)) of trichloroacetic acid_{(aq)} was added and mixing was left for at least 1hr*. Alternative amounts of trichloroacetic acid can be added during sample preparation and include e.g., at least 5%, at least 10%, at least 15%, at least 20%, at least 25% (w/v), or higher.

The use of trichloroacetic acid in the sample preparation was found to negate the need for ion pairing reagents to be used in the mobile phase of the LC system. This effectively removes the risk of contamination to the LC-MS/MS system when using such additives. In addition, when injecting a minimum of 10µL of a final diluent concentration of ≥50g/L (≥5% (w/v)) of trichloroacetic acid, equating to an on column concentration of ≥0.5mg, the negatively charged portion of the acid may act by binding to the positively charged sites in the column and system flow path (Fe⁺ and silanol groups). As a result, there were fewer charged sites remaining in the analytical system flow path and therefore the negatively charged phosphate groups present on thiamine pyrophosphate and pyridoxal 5-phosphate do not interact, preventing any unwanted secondary interactions during chromatographic separation.

Once mixing was complete, the sample was centrifuged at 5,000g for 5min at 4°C.

The samples were transferred into a 1mL square collection plate for injection on the UPLC/MS/MS (Needle placement 2mm) or were injected directly off the pellet (Needle placement 10mm). In one alternative, the supernatant from the mixed sample was transferred into a 1mL round 96 well collection plate for injection on the UPLC/MS/MS.

*The mixing times were not optimized after the sample was added to the internal standard and after the trichloroacetic acid was added for the current method. A time course experiment could be performed to possibly reduce the mixing times.

### Results

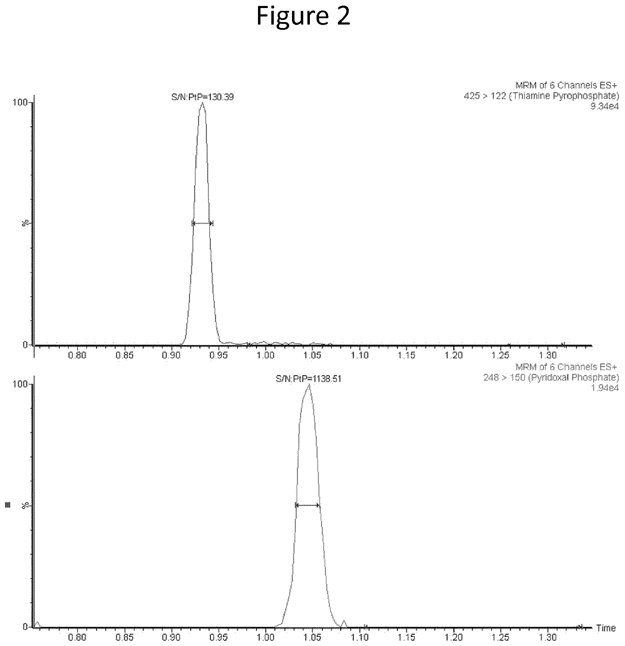

The chromatograms in **Figure 1** show the results of a low level sample: TPP approx 70nmol/L, PLP approx 45nmol/L. As shown by **Figure 2****,** a good signal to noise ratio was obtained from the low patient samples analyzed. This is due to the low amount of noise seen within all samples analyzed. TPP S:N ratio = 125 (peak to peak) at 70nmol/L PLP S:N ratio = 1019 (peak to peak) at 45nmol/L.

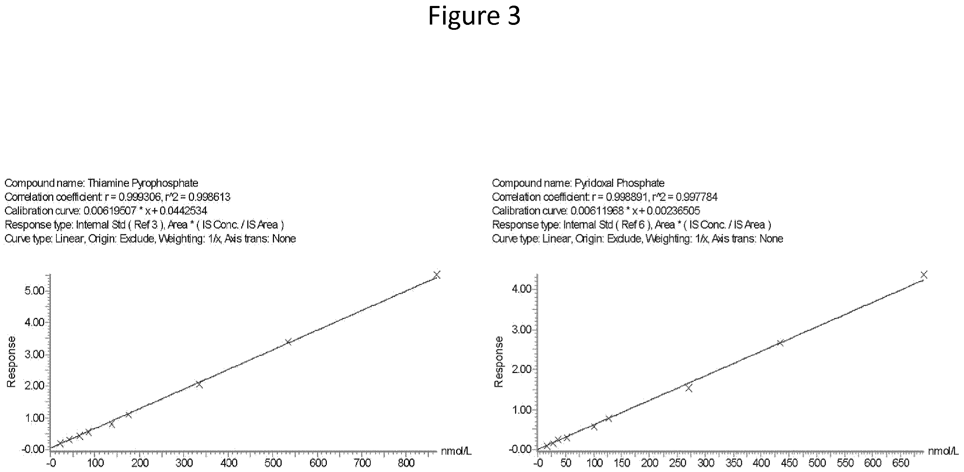

Calibration lines are shown in **Figure 3****.** Good r² values (linearity) were obtained from whole blood calibrators (also diluted in PBS/BSA to obtain concentrations lower than approx 100nmol/L). The following calibration ranges were used in the 5 day imprecision run. TPP: 22.5 - 867.7nmol/L. PLP: 15.1 - 691.3nmol/L. In some instances, the gradient of the whole blood PLP calibration line reduced over the course of the 5 day imprecision testing. Without wishing to be bound by theory, it is thought that this could be due to a stability issue, either with freeze/thaw cycling or by storing the samples at ambient temperature while performing the sample preparation. PBS/BSA calibrators were also run alongside during testing which showed a more consistent gradient, therefore these were used during the processing of the imprecision data for PLP.

### Imprecision

Samples from 4 different individuals to best span the concentration range were selected. 5 preparations of each individual were prepared and run on each of 5 days to assess imprecision. Good total run imprecision and repeatability were obtained for TPP and PLP. Imprecision data of ≤5.3% and 9.6% respectively, for all patient concentration levels, were observed.

**TPP - Whole Blood Calibrators**

| **Sample** | **Mean** | **Repeatability** | **Total** |
|---|---|---|---|
| | **(nmol/L)** | **(%)** | **(%)** |
| **Patient A** | 73.3 | 5.3 | 5.3 |
| **Patient B** | 85.3 | 4.1 | 4.9 |
| **Patient C** | 114.4 | 4.5 | 4.6 |
| **Patient D** | 206.1 | 4.0 | 5.2 |

**PLP - PBS/BSA Calibrators**

| **Sample** | **Mean** | **Repeatability** | **Total** |
|---|---|---|---|
| | **(nmol/L)** | **(%)** | **(%)** |
| **Patient A** | 44.5 | 9.3 | 9.6 |
| **Patient B** | 87.2 | 6.1 | 7.0 |
| **Patient C** | 140.5 | 4.1 | 4.1 |
| **Patient D** | 307.8 | 5.6 | 6.3 |

Unless
otherwise defined, all technical and scientific terms used herein are accorded the meaning commonly known to one with ordinary skill in the art.

When used in this specification and claims, the terms "comprises" and "comprising" and variations thereof mean that the specified features, steps or integers are included. The terms are not to be interpreted to exclude the presence of other features, steps or components.

## Claims

1. A method of quantifying both native Thiamine Pyrophosphate (TPP) and native Pyridoxal 5-Phosphate (PLP) from a single sample of human whole blood using mass spectrometry, comprising:
mixing the single sample of human whole blood with 20% (w/v) trichloroacetic acid;
eluting TPP and PLP from a reverse phase C18 high performance liquid chromatography column using a mobile phase comprising water, methanol and formic acid;
generating a mass spectrometry signal during elution of the TPP and PLP; and
quantifying the TPP and PLP using one or more calibration standards;
wherein the TPP and the PLP are eluted at a time of less than 20 seconds apart from one another and wherein the total run time is 3.5 minutes or less.

2. The method of Claim 1, wherein quantifying the TPP and PLP using one or more calibration standards comprises augmenting human whole blood with TPP and/or PLP, and optionally diluting the human whole blood with phosphate buffered saline to generate the one or more calibration standards over a measuring range to which the amount of the TPP and PLP eluted from the column can be determined by mathematical expression.

3. The method of Claim 1, wherein quantifying the TPP and PLP using one or more calibration standards comprises augmenting TPP and PLP depleted whole blood with TPP and PLP to generate the one or more calibration standards over a measuring range to which the amount of the TPP and PLP eluted from the column can be determined by mathematical expression.

4. The method of Claim 1, wherein quantifying the TPP and PLP using one or more calibration standards comprises the use of surrogate matrix calibrators prepared from phosphate buffered saline solution optionally containing human serum albumin or bovine serum albumin and optionally augmented with TPP and PLP to generate the one or more calibration standards over a measuring range to which the amount of the TPP and PLP eluted from the column can be determined by mathematical expression.

5. The method of Claim 1, wherein quantifying the TPP and PLP using one or more calibration standards comprises the use of surrogate analyte calibrators prepared in human whole blood by the addition of a different stable labeled isotope to that of the internal standard for TPP and PLP to generate the one or more calibration standards over a measuring range to which the amount of the TPP and PLP eluted from the column can be determined by mathematical expression.

6. The method of Claim 1, wherein quantifying the TPP and PLP using one or more calibration standards comprises the use placing one or more calibration standards in the same sample together with the TPP and PLP eluted from the column, to which the amount of the TPP and PLP eluted from the column can be determined by mathematical expression.

7. The method of any one of Claims 1 to 6, wherein the one or more calibrators are provided in the form of a kit.

8. A method of separating both native Thiamine Pyrophosphate (TPP) and native Pyridoxal 5-Phosphate (PLP) from whole blood comprising mixing the single sample of human whole blood with 20% (w/v) trichloroacetic acide, eluting TPP and PLP at time of less than 20 seconds apart from one another from a reverse phase C18 high performance liquid chromatography column using a mobile phase comprising water, methanol and formic acid, wherein the total run time is 3.5 minutes or less.

9. The method of any one of Claims 1 to 8, comprising eluting TPP and PLP at time of less than 17 seconds apart from one another from the liquid chromatography column, preferably less than 15 seconds apart from one another from the liquid chromatography column, preferably less than 13 seconds apart from one another from the liquid chromatography column.

10. The method of any one of Claims 1 to 9, wherein both the TPP and PLP have a retention time on the liquid chromatography column of 35 seconds or greater, preferably 40 seconds or greater, preferably 45 seconds or greater.

11. The method of any one of Claims 1 to 10, wherein the total elution time of TPP and PLP does not exceed 3.0 minutes, preferably wherein the total elution time of TPP and PLP does not exceed 2.5 minutes, preferably wherein the total elution time of TPP and PLP does not exceed 2.0 minutes.

12. The method of any one of Claims 1 to 11, wherein the liquid chromatography column is a reverse phase C18 HPLC column, having a 3.5 µm particle size, or wherein the liquid chromatography column is a reverse phase C18 HPLC column having a 3.5 µm particle size, a greater than 15% carbon load, preferably a 15% to 20% carbon load, and a pore size of greater than or equal to 100 Å.

13. The method of any one of Claims 1 to 12, wherein the TPP and PLP are eluted from the liquid chromatography column using a mobile phase comprising H₂O together with 0.1% formic acid (v/v) and MeOH together with 0.1% formic acid (v/v), or wherein the TPP and PLP are eluted from the liquid chromatography column using H₂O together with 0.1% formic acid (v/v) and MeOH together with 0.1% formic acid (v/v), subject to a gradient comprising the following conditions:
| Time (min) | Flow Rate (mL/min) | % Total of H₂O together with 0.1% formic acid (v/v) | % Total of MeOH together with 0.1% formic acid (v/v) | Curve |
|---|---|---|---|---|
| 0 | 0.60 | 97 | 3 | Initial |
| 0.60 | 0.60 | 70 | 30 | 6 |
| 1.20 | 0.80 | 3 | 97 | 11 |
| 1.70 | 0.80 | 97 | 3 | 11 |
| 1.90 | 0.60 | 97 | 3 | 11 |

14. The method of any one of Claims 1 to 13, further comprising the step of protein precipitating the TPP and PLP from whole blood prior to elution on the liquid chromatography column.

## Patentansprüche

1. Verfahren zur Quantifizierung von sowohl nativem Thiaminpyrophosphat (TPP) als auch nativem Pyroxidal-5-phosphat (PLP) aus einer einzigen Probe von menschlichem Vollblut unter Verwendung von Massenspektrometrie, umfassend:
Mischen der einzigen Probe von menschlichem Vollblut mit 20%-iger (w/v) Trichloressigsäure;
Elutieren von TPP und PLP aus einer Umkehrphasen-C18-Hochdruckflüssigkeitschromatographiesäule unter Verwendung einer mobilen Phase, die Wasser, Methanol und Ameisensäure umfasst;
Erzeugen eines Massenspektrometriesignals während der Elution von TPP und PLP und
Quantifizieren des TPP und des PLP unter Verwendung von einem oder mehreren Kalibrierstandards;
wobei das TPP und das PLP in einer Zeit von weniger als 20 Sekunden voneinander getrennt elutiert werden und wobei die Gesamtlaufzeit 3,5 Minuten oder kürzer ist.

2. Verfahren nach Anspruch 1, wobei das Quantifizieren des TPP und des PLP unter Verwendung von einem oder mehreren Kalibrierstandards ein Anreichern von menschlichem Vollblut mit TPP und/oder PLP und optional ein Verdünnen des menschlichen Vollbluts mit phosphatgepufferter Kochsalzlösung umfasst, um den einen oder die mehreren Kalibrierstandards über einen Messbereich zu erzeugen, woraus die Menge des aus der Säule elutierten TPP und PLP durch mathematischen Ausdruck bestimmt werden kann.

3. Verfahren nach Anspruch 1, wobei das Quantifizieren des TPP und des PLP unter Verwendung von einem oder mehreren Kalibrierstandards ein Anreichern von TPP- und PLP-abgereichertem Vollblut mit TPP und PLP umfasst, um den einen oder die mehreren Kalibrierstandards über einen Messbereich zu erzeugen, woraus die Menge des aus der Säule elutierten TPP und PLP durch mathematischen Ausdruck bestimmt werden kann.

4. Verfahren nach Anspruch 1, wobei das Quantifizieren des TPP und des PLP unter Verwendung von einem oder mehreren Kalibrierstandards die Verwendung von Surrogatmatrixkalibratoren umfasst, die aus phosphatgepufferter Kochsalzlösung hergestellt sind, die optional menschliches Serumalbumin oder Rinderserumalbumin enthält und optional mit TPP und PLP angereichert ist, um den einen oder die mehreren Kalibrierstandards über einen Messbereich zu erzeugen, woraus die Menge des aus der Säule elutierten TPP und PLP durch mathematischen Ausdruck bestimmt werden kann.

5. Verfahren nach Anspruch 1, wobei das Quantifizieren des TPP und des PLP unter Verwendung von einem oder mehreren Kalibrierstandards die Verwendung von Surrogatanalytkalibratoren umfasst, die in menschlichem Vollblut durch die Zugabe eines anderen stabil markierten Isotops zu dem des internen Standards für TLP und PLP hergestellt sind, um den einen oder die mehreren Kalibrierstandards über einen Messbereich zu erzeugen, woraus die Menge des aus der Säule elutierten TPP und PLP durch mathematischen Ausdruck bestimmt werden kann.

6. Verfahren nach Anspruch 1, wobei das Quantifizieren des TPP und des PLP unter Verwendung von einem oder mehreren Kalibrierstandards die Verwendung eines Gebens von einem oder mehreren Kalibrierstandards in dieselbe Probe zusammen mit dem aus der Säule elutierten TPP und PLP umfasst, woraus die Menge des aus der Säule elutierten TPP und PLP durch mathematischen Ausdruck bestimmt werden kann.

7. Verfahren nach einem der Ansprüche 1 bis 6, wobei der eine oder die mehreren Kalibratoren in der Form eines Kits bereitgestellt sind.

8. Verfahren zur Trennung von sowohl nativem Thiaminpyrophosphat (TPP) als auch nativem Pyroxidal-5-phosphat (PLP) aus Vollblut, umfassend ein Mischen der einzigen Probe von menschlichem Vollblut mit 20%-iger (w/v) Trichloressigsäure, ein Elutieren von TPP und PLP in einer Zeit von weniger als 20 Sekunden voneinander getrennt aus einer Umkehrphasen-C18-Hochdruckflüssigkeitschromatographiesäule unter Verwendung einer mobilen Phase, die Wasser, Methanol und Ameisensäure umfasst, wobei die Gesamtlaufzeit 3,5 Minuten oder kürzer ist.

9. Verfahren nach einem der Ansprüche 1 bis 8, umfassend ein Elutieren von TPP und PLP in einer Zeit von weniger als 17 Sekunden voneinander getrennt aus der Flüssigkeitschromatographiesäule, vorzugsweise weniger als 15 Sekunden voneinander getrennt aus der Flüssigkeitschromatographiesäule, vorzugsweise weniger als 13 Sekunden voneinander getrennt aus der Flüssigkeitschromatographiesäule.

10. Verfahren nach einem der Ansprüche 1 bis 9, wobei sowohl das TPP als auch das PLP eine Retentionszeit auf der Flüssigkeitschromatographiesäule von 35 Sekunden oder länger, vorzugsweise 40 Sekunden oder länger, vorzugsweise 45 Sekunden oder länger aufweisen.

11. Verfahren nach einem der Ansprüche 1 bis 10, wobei die Gesamtelutionszeit von TPP und PLP 3,0 Minuten nicht überschreitet, vorzugsweise wobei die Gesamtelutionszeit von TPP und PLP 2,5 Minuten nicht überschreitet, vorzugsweise die Gesamtelutionszeit von TPP und PLP 2,0 Minuten nicht überschreitet.

12. Verfahren nach einem der Ansprüche 1 bis 11, wobei die Flüssigkeitschromatographiesäule eine Umkehrphasen-C18-HPLC-Säule mit einer Teilchengröße von 3,5 µm ist oder wobei die Flüssigkeitschromatographiesäule eine Umkehrphasen-C18-HPLC-Säule mit einer Teilchengröße von 3,5 µm ist, einer Kohlenstoffbeladung von mehr als 15 %, vorzugsweise einer Kohlenstoffbeladung von 15 % bis 20 % und einer Porengröße von größer gleich 100 Å ist.

13. Verfahren nach einem der Ansprüche 1 bis 12, wobei das TPP und das PLP aus der Flüssigkeitschromatographiesäule unter Verwendung einer mobilen Phase, die H₂O zusammen mit 0,1%-iger Ameisensäure (v/v) und MeOH zusammen mit 0,1%-iger Ameisensäure (v/v) umfasst, elutiert werden oder wobei das TPP und das PLP aus der Flüssigkeitschromatographiesäule unter Verwendung von H₂O zusammen mit 0,1%-iger Ameisensäure (v/v) und MeOH zusammen mit 0,1%-iger Ameisensäure (v/v) elutiert werden, wobei sie einem Gradienten unterzogen werden, der die folgenden Bedingungen umfasst:
| Zeit (min) | Flussrate (mL/min) | % von H₂O zusammen mit 0,1%-iger Ameisensäure (v/v) insgesamt | % von MeOH zusammen mit 0,1%-iger Ameisensäure (v/v) insgesamt | Kurve |
|---|---|---|---|---|
| 0 | 0, 60 | 97 | 3 | Anfangs |
| 0,60 | 0, 60 | 70 | 30 | 6 |
| 1,20 | 0,80 | 3 | 97 | 11 |
| 1,70 | 0,80 | 97 | 3 | 11 |
| 1,90 | 0,60 | 97 | 3 | 11 |

14. Verfahren nach einem der Ansprüche 1 bis 13, weiterhin umfassend den Schritt eines Proteinpräzipitierens des TPP und des PLP aus Vollblut vor der Elution auf der Flüssigkeitschromatographiesäule.

## Revendications

1. Procédé de quantification à la fois de Thiamine Pyrophosphate (TPP) natif et de Pyridoxal 5-Phosphate (PLP) natif à partir d'un échantillon unique de sang total humain à l'aide de la spectrométrie de masse, comprenant :
mélanger l'échantillon unique de sang total humain avec 20 % (p/v) d'acide trichloroacétique ;
éluer les TPP et PLP à partir d'une colonne de chromatographie liquide haute performance C18 en phase inverse à l'aide d'une phase mobile comprenant de l'eau, du méthanol et de l'acide formique ;
générer un signal de spectrométrie de masse pendant l'élution des TPP et PLP ; et
quantifier les TPP et PLP à l'aide d'un ou plusieurs standards d'étalonnage ;
dans lequel le TPP et le PLP sont élués à des temps séparés de moins de 20 secondes l'un de l'autre et dans lequel le temps de passage total est de 3,5 minutes ou moins.

2. Procédé selon la revendication 1, dans lequel quantifier les TPP et PLP à l'aide d'un ou plusieurs standards d'étalonnage comprend enrichir le sang total humain par du TPP et/ou du PLP, et facultativement diluer le sang total humain avec une solution saline tamponnée au phosphate pour générer le ou les standards d'étalonnage sur une plage de mesure à laquelle la quantité des TPP et PLP élués à partir de la colonne peut être déterminée par expression mathématique.

3. Procédé selon la revendication 1, dans lequel quantifier les TPP et PLP à l'aide d'un ou plusieurs standards d'étalonnage comprend enrichir le sang total appauvri en TPP et PLP par du TPP et du PLP pour générer le ou les standards d'étalonnage sur une plage de mesure à laquelle la quantité des TPP et PLP élués à partir de la colonne peut être déterminée par expression mathématique.

4. Procédé selon la revendication 1, dans lequel quantifier les TPP et PLP à l'aide d'un ou plusieurs standards d'étalonnage comprend l'utilisation d'étalonneurs de matrice de substitution préparés à partir de solution saline tamponnée au phosphate contenant facultativement de la sérum albumine humaine ou de la sérum albumine bovine et facultativement enrichie par du TPP et du PLP pour générer le ou les standards d'étalonnage sur une plage de mesure à laquelle la quantité des TPP et PLP élués à partir de la colonne peut être déterminée par expression mathématique.

5. Procédé selon la revendication 1, dans lequel quantifier les TPP et PLP à l'aide d'un ou plusieurs standards d'étalonnage comprend l'utilisation d'étalonneurs d'analyte de substitution préparés dans du sang total humain par l'addition d'un isotope marqué stable différent de celui du standard interne pour les TPP et PLP pour générer le ou les standards d'étalonnage sur une plage de mesure à laquelle la quantité des TPP et PLP élués à partir de la colonne peut être déterminée par expression mathématique.

6. Procédé selon la revendication 1, dans lequel quantifier les TPP et PLP à l'aide d'un ou plusieurs standards d'étalonnage comprend placer un ou plusieurs standards d'étalonnage dans le même échantillon conjointement avec les TPP et PLP élués à partir de la colonne, la quantité des TPP et PLP élués à partir de la colonne peut être déterminée par expression mathématique.

7. Procédé selon l'une quelconque des revendications 1 à 6, dans lequel le ou les étalonneurs sont fournis sous la forme d'un kit.

8. Procédé de séparation à la fois du Thiamine Pyrophosphate (TPP) natif et du Pyridoxal 5-Phosphate (PLP) natif à partir de sang total comprenant : mélanger l'échantillon unique de sang total humain avec 20 % (p/v) d'acide trichloroacétique, éluer les TPP et PLP à des temps séparés de moins de 20 secondes l'un de l'autre à partir d'une colonne de chromatographie liquide haute performance C18 en phase inverse à l'aide d'une phase mobile comprenant de l'eau, du méthanol et de l'acide formique, le temps de passage total étant de 3,5 minutes ou moins.

9. Procédé selon l'une quelconque des revendications 1 à 8, comprenant : éluer les TPP et PLP à des temps séparés de moins de 17 secondes l'un de l'autre à partir de la colonne de chromatographie liquide, de préférence séparés de moins de 15 secondes l'un de l'autre à partir de la colonne de chromatographie liquide, de préférence séparés de moins de 13 secondes l'un de l'autre à partir de la colonne de chromatographie liquide.

10. Procédé selon l'une quelconque des revendications 1 à 9, dans lequel à la fois le TPP et le PLP ont un temps de rétention sur la colonne de chromatographie liquide de 35 secondes ou plus, de préférence de 40 secondes ou plus, de préférence de 45 secondes ou plus.

11. Procédé selon l'une quelconque des revendications 1 à 10, dans lequel le temps d'élution total de TPP et de PLP ne dépasse pas 3,0 minutes, de préférence dans lequel le temps d'élution total de TPP et de PLP ne dépasse pas 2,5 minutes, de préférence dans lequel le temps d'élution total de TPP et de PLP ne dépasse pas 2,0 minutes.

12. Procédé selon l'une quelconque des revendications 1 à 11, dans lequel la colonne de chromatographie liquide est une colonne de HPLC C18 en phase inverse, ayant une dimension de particule de 3,5 µm, ou dans lequel la colonne de chromatographie liquide est une colonne de HPLC C18 en phase inverse, ayant une dimension de particule de 3,5 pm, une charge en carbone de plus de 15 %, de préférence une charge en carbone de 15 % à 20 %, et une dimension de pore supérieure ou égale à 100 Å.

13. Procédé selon l'une quelconque des revendications 1 à 12, dans lequel les TPP et PLP sont élués à partir de la colonne de chromatographie liquide à l'aide d'une phase mobile comprenant H₂O conjointement avec 0,1 % d'acide formique (v/v) et MeOH conjointement avec 0,1 % d'acide formique (v/v), ou dans lequel les TPP et PLP sont élués à partir de la colonne de chromatographie liquide à l'aide de H₂O conjointement avec 0,1 % d'acide formique (v/v) et MeOH conjointement avec 0,1 % d'acide formique (v/v), soumis à un gradient comprenant les conditions suivantes :
| Temps (min) | Débit (mL/min) | % total de H₂O conjointement avec 0,1 % d'acide formique (v/v) | % total de MeOH conjointement avec 0,1 % d'acide formique (v/v) | Courbe |
|---|---|---|---|---|
| 0 | 0,60 | 97 | 3 | Initiale |
| 0,60 | 0,60 | 70 | 30 | 6 |
| 1,20 | 0,80 | 3 | 97 | 11 |
| 1,70 | 0,80 | 97 | 3 | 11 |
| 1,90 | 0,60 | 97 | 3 | 11 |

14. Procédé selon l'une quelconque des revendications 1 à 13, comprenant en outre l'étape de précipitation de protéines des TPP et PLP à partir du sang total avant l'élution sur la colonne de chromatographie liquide.
